# EUROPEAN PATENT APPLICATION

(11) **EP 3 674 393 A1**
(43) Date of publication of application: **01.07.2020**
(21) Application number: 18872941.2
(22) Date of filing: 03.11.2018
(51) Int. Cl.: C12M 1/38, C12M 1/34

(54) **DROPLET DETECTION APPARATUS**

(30) Priority: 06.11.2017 CN 201711074957; 06.11.2017 CN 201711074983; 06.11.2017 CN 201711074989
(71) Applicant: Targetingone Corporation, Beijing 100190 (CN)
(72) Inventor: JING, Gaoshan, Beijing 100190 (CN); GUO, Yong, Beijing 100190 (CN); WANG, Bo, Beijing 100190 (CN); SU, Shisheng, Beijing 100190 (CN); BAI, Yu, Beijing 100190 (CN); ZHU, Xiurui, Beijing 100190 (CN); FU, Mingzhu, Beijing 100190 (CN); ZHU, Lingxiang, Beijing 100190 (CN); LIU, Baoxia, Beijing 100190 (CN); YANG, Wenjun, Beijing 100190 (CN); WANG, Yongdou, Beijing 100190 (CN)
(74) Representative: LLR
(86) International application number: PCT/CN2018/113852
(87) International publication number: WO 2019/086019

(57) **Abstract**

A droplet detection apparatus, comprising a first component (1), a second component (2) and a third component (3), the first component (1) is a component used for loading a spacer oil and a floating oil when detecting droplets and collecting a waste liquid after detection, the second component (2) is a droplet detection chip, the third component (3) is a container holding droplets for droplet PCR amplification, the second component (2) is fixedly connected to an upper surface of the first component (1), the third component (3) is detachably fixedly connected to a lower surface of the first component (1). The droplet detection apparatus detects fluorescence signals of droplet samples in a rapid, reliable, convenient and contamination-free manner. The droplet detection apparatus has low material and processing costs and is beneficial for a wide range of clinical tests and other biological tests.

## Description

### Technical Field

The invention relates to the technical field of droplet digital PCR, in particular to a droplet detection apparatus.

### Background

Droplet digital PCR (ddPCR) is an absolute quantitative analysis technology of nucleic acid based on single-molecule PCR. Droplet digital PCR technology is becoming the next revolutionary technology in the industry with its advantages of high sensitivity and high accuracy. In recent years, with the development of micro-nanofabrication and microfluidics, droplet PCR technology has encountered the best opportunity to break through the technical bottleneck. This technology uses microfluidic chips to generate droplets with diameters ranging from several micrometers to hundreds of micrometers; droplets encapsulate single molecules or single cells to achieve complete sealing and integration of reaction and detection. The working principle of the droplet digital PCR system is: firstly, the sample to be measured is evenly divided into a large number of nano-upgraded (a few micrometers to hundreds of micrometers) droplets by a special droplet generator. The number of droplets is in the millions. Because the number of droplets is sufficient and the droplets are isolated from each other by the oil layer, each droplet is equivalent to a "microreactor", and the droplets only contain a single DNA molecule of the sample to be tested; then, these droplets are transferred to EP tubes and reacted in a conventional PCR machine. After the PCR amplification reaction of the droplets, the fluorescence signals of the droplets are detected one by one by a special droplet analyzer. The droplets with a fluorescence signal are interpreted as 1, and the droplets without a fluorescence signal are interpreted as 0. Finally, according to the Poisson distribution principle and the number and proportion of positive droplets, the number of target DNA molecules of the sample to be measured can be obtained, and the absolute quantification of the nucleic acid sample can be achieved. A key step of the droplet digital PCR technology is to quickly and reliably determine the fluorescence signal of the droplet sample and identify the number and proportion of positive droplets.

The determination of the fluorescence signal of a droplet sample depends on a core technology: the design and processing of a droplet fluorescence detection apparatus, which uses laser to excite the fluorescence signal of the product in the droplet to distinguish between negative droplets and positive droplets. The general procedure of droplet digital PCR technology is: the generated droplets are transferred to an EP tube and reacted in a conventional PCR instrument. The droplets subjected to the PCR amplification reaction are injected into a droplet fluorescence detection apparatus, and a special droplet analyzer is used for fluorescence signal detection. The droplet fluorescence detection apparatus is widely used and clinical detection needs to have the following principles: (1) an EP tube containing droplets undergoing PCR amplification reaction is tightly connected to the droplet fluorescence detection apparatus, without transfer, reducing possible cross-contamination; (2) the droplets are injected into the droplet fluorescence detection apparatus under the action of a special droplet analyzer; when the droplets flow through the laser detection area, a single row is neatly arranged, which is convenient for accurate detection of fluorescent signals; (3) the droplets after detection are collected in a closed storage container to reduce possible cross-contamination; (4) the droplet fluorescence detection apparatus is used once, material and processing costs are Low, (5) Convenient operation of the droplet fluorescence detection apparatus. In view of the above principles, the droplet detection chip based on micro-fluidic technology has great application prospects.

At present, microfluidic chips based on polydimethylsiloxane (PDMS) have been widely used to detect droplets. First, the researchers used a soft lithography process (manual operation) to process PDMS droplet chips with micrometer-scale. After the PDMS droplet chip is successfully prepared, the sample inlet and the droplet generation outlet are punched by mechanical processing, and the sample inlet tube and the sample outlet tube are assembled. The "oil phase" sample and the "droplet" sample in the EP tube were manually sucked into the syringe. Then, the "oil phase" sample and the "droplet" sample were injected into the PDMS droplet chip through an injection tube through an external syringe pump. In the pre-designed flow channel area, the optical detection system detects the fluorescence signal of the droplet one by one. Finally, the detected droplets are collected into a conventional experimental consumable through a sample tube, such as an EP tube. Although the PDMS droplet chip material has low research and development costs and simple laboratory processing technology, its shortcomings include:
(1) The PDMS droplet detection chip is open-connected to an EP tube containing a "droplet" sample, which easily causes cross-contamination.
(2)The detected droplets are collected in an open EP tube, which is likely to cause cross-contamination.
(3) PDMS is a thermoelastic polymer material, which is not suitable for industrial-grade injection molding and packaging processes. Manually processed PDMS droplet chips have poor reliability.
   PDMS droplet chip batch processing is expensive.
(4)PDMS droplet chip sample injection and droplet collection are cumbersome manual operation procedures, and are not suitable for clinical testing applications.

Aiming at the shortage of PDMS droplet chip, we designed and processed a droplet detection apparatus based on micro-fluidic technology.

The droplet detection apparatus can detect the fluorescence signal of a droplet sample quickly, reliably, conveniently, and without pollution; the material and processing cost of the droplet detection apparatus are low, which is beneficial to the widespread application of clinical detection.

### Summary of the Invention

In one embodiment, the present invention provides a droplet detection apparatus. The droplet detection apparatus comprises a first component, a second component, and a third component, said first component is the component used for loading spacer oil and floating oil when detecting droplets and collecting waste liquid after the detection of the droplets, said second component is a droplet detection chip, and said third component is a container holding droplets for droplet PCR amplification; and said second component is fixedly connected to the upper surface of said first component; and said third component is detachably fixedly connected to the lower surface of said first component..

In one embodiment, said first component and said second component are respectively formed by integral injection molding.

In one embodiment, said first component is provided with a locking slot for fixing said third component.

In one embodiment, said third component comprises a droplet container and a cover of said droplet container, said droplet container is made of a rigid plastic material, and said cover is made of a soft plastic material; when fixing, said cover cooperates with said locking slot so that said first component and said third component are detachably fixedly connected .

In one embodiment, said locking slot is provided with a puncture needle protruding from the lower surface of said first component, and said puncture needle is hollow; and when said first component and said third component are detachably fixedly connected, said puncture needle penetrates the cover of said container.

In one embodiment, said first component comprises at least one unit for loading spacer oil and floating oil when detecting droplets and collecting waste liquid after the detection of the droplets, preferably four, eight or twelve; said second component comprises at least one droplet detection unit, preferably four, eight or twelve; said third component comprises at least one said container, preferably four, eight, or twelve; one said unit for loading spacer oil and floating oil when detecting droplets and collecting waste liquid after the detection of the droplets, one said droplet detection unit and one said container holding droplets for droplet PCR amplification are matched to finish the detection of the droplets after PCR amplification.

In one embodiment, said unit for loading spacer oil and floating oil when detecting droplets and collecting waste liquid after the detection of the droplets comprises a floating oil loading tank, a floating oil loading through hole, a spacer oil loading tank and a spacer oil loading through hole provided on the upper end of the upper surface of said first component; said floating oil loading through hole and said spacer oil loading through hole are respectively provided at the bottom of said floating oil loading tank and said spacer oil loading tank; and said floating oil loading through hole is connected to a floating oil injection conduit at the bottom of the lower surface of said first component, said spacer oil loading through hole is connected to a spacer oil injection conduit at the bottom of the lower surface of said first component.

In one embodiment, a waste liquid tank is provided on the lower end of the lower surface of said first component and is used to collect all waste liquid after detection.

In one embodiment, a configuration for discharging residual air in said waste liquid tank is provided on the lower end of said second component.

In one embodiment, said droplet detection chip comprises one central hole, said central hole is used for injecting molding materials during the preparation of said droplet detection chip and used for transport of droplet detection chips during mass production of said droplet detection chips and/or mass fluorescence detection of said droplet detection chips; and one or more droplet detection units are provided on both sides of said central hole, each droplet detection unit independently detects droplets.

In one embodiment, said droplet detection unit comprises a droplet detection conduit, a spacer oil inlet, a spacer oil conduit, a droplet container, a droplet floating hole and a droplet conduit; spacer oil enters said spacer oil conduit from said spacer oil inlet, and droplets enter said droplet conduit from said droplet container through said droplet floating hole; said spacer oil conduit and said droplet conduit form a cross structure before said droplet detection conduit, so that the droplets in said droplet conduit are separated by spacer oil.

In one embodiment, a return flow resistance zone is provided in the conduit after said spacer oil inlet, and spacer oil is divided into two paths to enter two said spacer oil conduits after passing through said return flow resistance zone, respectively.

In one embodiment, a spacer oil filter zone is provided in said spacer oil conduit and/or a floating oil filter zone is provided in said floating oil conduit, and said spacer oil filter zone and/or said floating oil filter zone are a set of columnar array structures, respectively.

In one embodiment, said spacer oil conduit and / or said droplet conduit are arc-shaped conduit structures far from said central hole; an auxiliary channel is provided beside said droplet detection conduit, so that the optical path of detection system is positioned to the center of said droplet detection conduit.

In one embodiment, said first component and said second component are thermoplastic materials, preferably polycarbonate materials, cycloolefin copolymers or polymethylmethacrylate, polypropylene.

In one embodiment, the volumes of said floating oil loading tank and said spacer oil loading tank are 1 to 900 microliters, preferably 5 to 500 microliters, and more preferably 100 to 200 microliters.

In one embodiment, an observation window is further provided on said second component, which is used to cooperate with the optical system to monitor the generated droplets in real time.

In one embodiment, the droplet detection apparatus further comprises a fourth component, and external pressure is applied to the spacer oil and the floating oil in the first component by the fourth component.

In one embodiment, positioning holes are provided on said first component and said second component to facilitate positioning of the two components.

In one embodiment, said first component and said second component are sealed and connected by a dispensing method or an ultrasonic welding method.

In one embodiment, the cross structure is formed by two said spacer oil conduits and one said droplet conduit.

In one embodiment, said droplet detection apparatus further includes a fourth component, and external pressure is applied to the spacer oil and the floating oil in said first component by the fourth component.

The droplet detection apparatus of the present invention can achieve the following effects:
(1) fast, reliable, and parallel detection of fluorescence signals of micron-level "water-in-oil" droplet samples.
(2)The first component and the second component of the apparatus are processed by using thermoplastic materials (such as PC, COP, PMMA), and the cost of materials and batch processing is low.
(3) In conjunction with an external pressure source, using the droplet detection apparatus, droplets The detection rate of droplet samples can be controlled at high speed.
(4)Integrated droplet chip design, the whole process is not prone to cross-contamination. The EP tube containing the droplet samples after PCR is directly connected to the chip, and there is no need to open the cover. The detected waste liquid will be collected in the waste liquid collection tank, and there is no cross-contamination in the whole process.
(5)The droplet fluorescence detection chip is easy to operate. It only needs to manually preload spacer oil and floating oil, and then cooperate with the optical detection instrument to complete the entire testing process conveniently.

In addition, the present invention proposes a droplet detection chip based on a polymer material. The droplet chip combines the industry's mature optical disc preparation process and optical disc design specifications, and uses innovative chip processing methods and chip designs. Its characteristics are: (1) droplets are arranged in a single row at regular intervals in the chip. Accurate detection of the fluorescence signal of the droplet sample after passing through laser detection zone is achieved. (2) The droplet chip uses thermoplastic materials such as polycarbonate, cycloolefin copolymer, polymethyl methacrylate and polypropylene. And the cost of materials and batch processing is low. (3) the structure of the traditional circular disc is modified, the space of the disc is maximized, and the droplet fluorescence information is detected in parallel.

### BRIEF DESCRIPTION OF THE DRAWINGS

In order to explain the technical solutions in the embodiments of the present application more clearly, the drawings used in the embodiments will be briefly introduced below. Obviously, the drawings in the following description are just some of the embodiments described in the application. For those of ordinary skill in the art, other drawings can be obtained based on these drawings without paying creative work.
Fig.1 is a schematic structural diagram of a droplet detection apparatus according to an embodiment of the present invention;
Fig.2 is a schematic structural diagram of a first component according to an embodiment of the present invention;
Fig. 3 is a schematic structural diagram of a first component connected to a third component through a locking slot according to an embodiment of the present invention;
Fig.4 is a schematic structural diagram of a third component according to an embodiment of the present invention;
Fig.5 is a schematic structural diagram of a second component according to an embodiment of the present invention;
Fig. 6 is a schematic structural diagram of a second component according to another embodiment of the present invention;
Fig. 7 is a schematic diagram of spacer oil return flow resistance zone according to an embodiment of the present invention;
Fig. 8 is a schematic diagram of filter zone of spacer oil conduit according to an embodiment of the present invention;
Fig.9 is a schematic diagram of a cross structure according to an embodiment of the present invention; and
Fig. 10 is a schematic structural diagram of a detection conduit according to an embodiment of the present invention.

### Description of Embodiments

In order to enable those skilled in the art to better understand the technical solutions in the present application, the present invention will be further described below with reference to the embodiments. Obviously, the described embodiments are only a part of the embodiments of the application, not all of the embodiments. Based on the embodiments in this application, all other embodiments obtained by a person of ordinary skill in the art without creative works should fall within the protection scope of this application. The invention is further described below with reference to the drawings and embodiments.

### Example 1 Droplet detection apparatus of the present invention

### 1. Structure of droplet detection apparatus

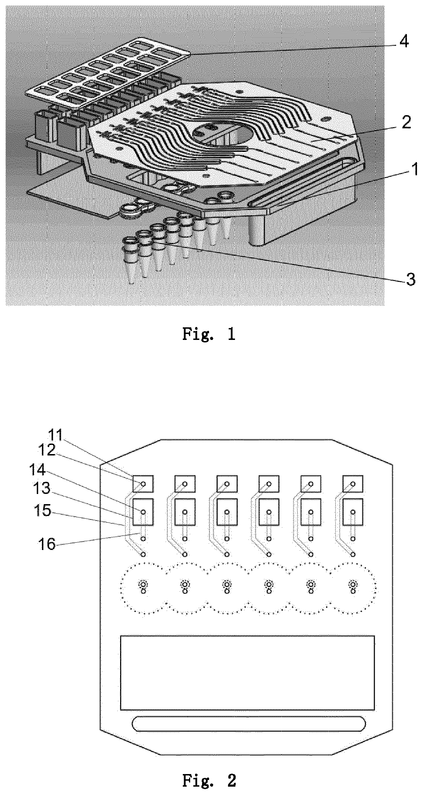

As shown in Fig. 1, the droplet detection apparatus of the present invention comprises a first component 1, a second component 2, and a third component 3. The first component 1 is used for loading spacer oil and floating oil when detecting droplets and collecting waste liquid after the detection of the droplets, the second component 2 is a droplet detection chip, and the third component 3 is a container holding droplets for droplet PCR amplification. The second component 2 is fixedly connected to the upper surface of the first component 1; and the third component 3 is detachably fixedly connected to the lower surface of the first component 1.

As shown in Fig. 1, in some embodiments, the droplet detection apparatus of the present invention further comprises a fourth component 4. The fourth component 4 is used to seal a floating oil loading tank and a spacer oil loading tank of the first component 1. The fourth component 4 is, for example, a water-sealing rubber gasket, for example, made of silica gel. The water-sealing rubber gasket may be provided with air holes for applying pressure therethrough, and its role is to ensure the pressure-tightness between the external pressure source and the first component, and is easy to apply external pressure.

As shown in Fig. 1, in some embodiments, positioning holes are processed in the first component and / or the second component 2 to facilitate cooperation with each other.

As shown in Fig. 2, The first component 1 is used for loading spacer oil and floating oil when detecting droplets and collecting waste liquid after the detection of the droplets. As shown in Fig. 2, in this embodiment, six units for loading spacer oil and floating oil when detecting droplets and collecting waste liquid after the detection of the droplets are arranged in parallel on the first component 1. From left to right, at upper end of the upper surface of the first component 1, each unit for loading spacer oil and floating oil when detecting droplets and collecting waste liquid after the detection of the droplets are arranged on the first component 1 in parallel at equal intervals, each unit for loading spacer oil and floating oil when detecting droplets and collecting waste liquid after the detection of the droplets cooperates with each droplet detection unit on the second component 2, the unit is used for loading spacer oil and floating oil when detecting droplets and collecting waste liquid after the detection of the droplets. At the upper end of upper surface of the first component 1 it is provided with a floating oil loading tank 11, a floating oil loading through hole 12, a spacer oil loading tank 13, and a spacer oil loading through hole 14, wherein the floating oil loading through hole 12 and the spacer oil loading through hole 14 are installed in the bottom center of the floating oil loading tank 11 and spacer oil loading tank 13, respectively; the floating oil loading through hole 12 is connected to a floating oil injection conduit 15 at the bottom of the lower surface of the first component 1, a spacer oil loading through hole 14 is connected to a spacer oil injection conduit 16 at the bottom of the lower surface of the first component 1.

In some embodiments, the floating oil injection conduit 15 and the spacer oil injection conduit 16 are processed from the lower surface of the first component 1 and then sealed with a sealing sheet to form a closed conduit.

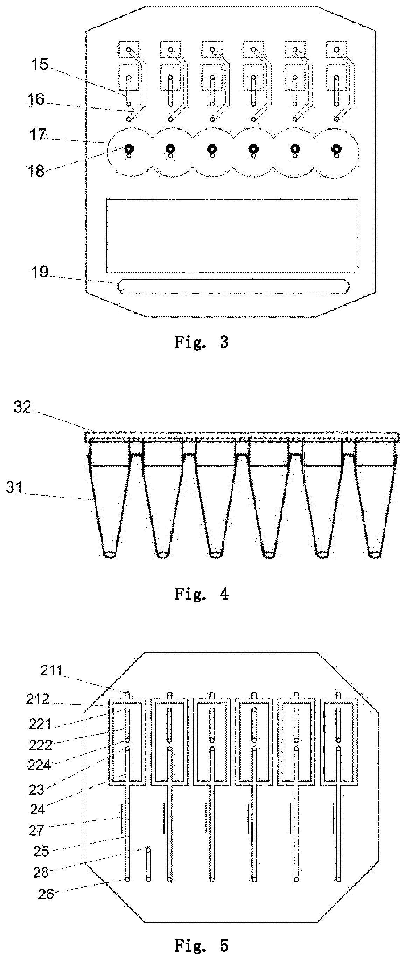

As shown in Fig. 3, on the lower surface of the first component 1, a locking slot 17 of the third component 3 is provided below the floating oil injection conduit 15 and the spacer oil injection conduit 16, and the locking slot 17 is detachably fixedly connected to the third component 3. The locking slot 17 is provided with a puncture needle 18 protruding from the lower surface of the first component 1, and the puncture needle 18 is hollow, through which the floating oil enters the third component.

A waste liquid tank 19 is provided on the lower end of the lower surface of the first component 1 and is used to collect all waste liquid after detection, so that the droplet after fluorescence detection is tightly recovered.

As shown in Fig. 4, the third component 3 includes a droplet container 31 for containing droplets and an upper cover 32 that can be punctured with a puncture needle 18 provided on the first component. The droplet container 31 is usually made of a rigid plastic material. For example, the droplet container 31 is a commonly used centrifuge tube (EP tube), and the upper cover 32 is usually made of a soft plastic material for easy puncture with a puncture needle 18, for example, the upper cover 32 is a cover of the centrifuge tube, and it is made of silicone rubber. In some embodiments, each droplet container 31 cooperates with each unit for loading spacer oil and floating oil when detecting droplets and collecting waste liquid after the detection of the droplets of the component 1. In some embodiments, each droplet container 31 is connected together. In this embodiment, the six droplet containers 31 are connected together, and the six upper covers 32 are connected together; they are each integrally processed.

As shown in Fig. 5, Fig. 5 is a schematic diagram of the droplet detection chip of the second component 2. Six droplet detection units are arranged in parallel on the second component 2, and from left to right each droplet detection unit is spaced in parallel. Each droplet detection unit cooperates with each unit for loading spacer oil and floating oil when detecting droplets and collecting waste liquid after the detection of the droplets of the component 1, which is used for detecting drops in parallel.

In some embodiments, 8, 10, 12, 14, 14, 16, 18, 20, 22, and 24 droplet detection units are arranged in parallel on the second component 2; a plurality of droplets are arranged in parallel on the second component 2, which mainly utilizes the area of the droplet detection chip to improve the detection efficiency.

As shown in Fig. 5, from top to bottom each droplet detection unit includes a spacer oil inlet 211, two spacer oil conduits 212, a floating oil inlet 221, a floating oil conduit 222, a floating oil hole 224, a droplet floating hole 23, a droplet conduit 24 and a droplet detection conduit 25 and waste liquid port 26.

In some embodiments, a positioning auxiliary channel 27 is provided close to the droplet detection conduit 25. The auxiliary channel 27 is a closed channel in which no oil phase, water phase, or droplet sample flows. This area has stable imaging and transmission/ astigmatism properties which facilitates the positioning of the optical path detection system to the center of the droplet detection conduit 25, helping to accurately find the area to be detected.

In some embodiments, an exhaust hole 28 is provided under the second component to exhaust the residual air in the waste liquid tank 19 when collecting the waste liquid. The exhaust hole 28 may or may not be a through hole on the second component, for example, residual air in the waste tank 19 is exhausted from the exhaust hole 28 through an exhaust pipe communicating with the atmosphere, one end of the exhaust pipe is an exhaust hole above the waste liquid tank, and the other end is a hole communicating with the atmosphere. The number of the exhaust holes 28 may be designed as required and may be one or plural. In order to prevent pollution, the upper surface of the hole communicated to the atmosphere can be sealed with a breathable film to reduce cross-contamination.

The first component 1 and the second component 2 are sealed and fixedly connected by a dispensing method or an ultrasonic welding method. For example, the first component 1 and the second component 2 are fixedly connected by a dispensing method around the floating oil inlet 221 and the spacer oil inlet 211 of the second component 2; the second component 2 is connected to the upper surface of the first component 1. The third component 3 is detachably and fixedly connected to the first component 1 through a locking slot 17 on the first component 1. In one embodiment, the third component 3 and the first component 1 are detachably and fixedly connected to the upper cover 32 through the locking slot 17, and the upper surface of the upper cover 32 is tightly connected to the lower surface of the locking slot 17 of the first component.

The floating oil loading through hole 12 of the first component 1 at the upper end of the lower surface of the first component 1 communicates with the floating oil inlet 221 of the second component through floating oil injection conduit 15; the spacer oil loading through hole 14 of the first component 1 at the upper end of the lower surface of the first component 1 communicates with the spacer oil inlet 211 of the second component through spacer oil injection conduit 16.

The floating oil and the spacer oil are loaded to the floating oil loading tank 11 and the spacer oil loading tank 13 of the first component 1, respectively. The external pressure source is connected through a fourth component 4, which is a sealing rubber pad accessory. The sealing rubber pad is silicone, and its role is to ensure the air tightness of the crimping between the external pressure source and the first component 1. Then, by means of external pressure, the floating oil flows into the floating oil injection conduit 15 through the floating oil loading through hole 12, then enters the floating oil conduit 222 through the floating oil inlet 221 on the second component 2, and finally enters the droplet container 31 through the floating oil hole 224 and the hollow of the puncture needle 18. The spacer oil flows into the spacer oil injection conduit 16 through the spacer loading through hole 14, and then enters the two spacer oil conduits 212 provided on the two sides of the spacer oil inlet 211 in parallel through the spacer oil inlet 211 on the second component 2.

The two spacer oil conduits 212 and the droplet conduit 24 form a cross structure before the droplet detection conduit 25. In the droplet detection conduit 25, the droplets are squeezed into a single row by the spacer oil to facilitate optical detection. When a single row of droplets passes through the optical detection zone, the fluorescence signal of each droplet is detected. Finally, the completed droplets are collected in a closed waste liquid tank 19.

In some embodiments, the first component 1 and the second component 2 are both prepared by a batch integrated injection molding process. In this way, the batch processing of the first component 1 and the second component 2 has high precision and good consistency, which is beneficial to the stable connection of the external pneumatic power apparatus. At the same time, the operation is simple and fast, and the results are stable. It is suitable for pushing the digital PCR technology based on droplet fluorescence detection to large-scale research and clinical testing applications.

### 2. Droplet Detection Process

The droplet detection process of the present invention is as follows. First, the generated droplets are collected into a droplet container 31, such as an EP tube. Then, the EP tube is equipped with a sealed EP tube cover 32 and placed in a conventional PCR instrument for PCR amplification reaction. Then, the droplet container 31 containing the amplified sample is assembled and connected with the first component 1 through the locking slot 17, and the puncture needle 18 completes the puncture of the upper cover 32 during the assembly and connection process.

The floating oil is preloaded in the floating oil loading tank 11 of the first component 1. Under the action of external pressure, the floating oil enters the floating oil conduit 222 from the floating oil inlet 221, and then enters the droplet container 31 through the floating oil hole 224 and the hollow of puncture needle 18. The first component 1 and the upper cover 32 of the third component 3 are hermetically connected, and the floating oil hole 224 is provided in the hollow corresponding to the puncture needle 18; the floating oil can flow into the droplet container 31 only from the hollow of the puncture needle 18. Since the density of the floating oil is greater than the density of the droplets in the droplet container 31, the droplets are always above the liquid surface.

During the puncture of the upper cover 32 by the puncture needle 18, a gap is formed around the puncture needle 18 passing through the upper cover 32, and the droplets in the droplet container 31 float up through the space around the puncture needle 18 under the action of the floating oil. Because the first component 1 and the second component 2 are closely connected, the floating droplets can only enter the droplet floating holes 23 on the second component 2 and then enter the droplet conduit 24.

As shown in Fig. 5, two spacer oil conduits 212 and one droplet conduit 24 form a cross structure, the purpose is to make the closely packed droplets floating up to be separated by the spacer oil on both sides, and to reduce the signal interference between the droplets. At the same time, the "cross structure" design can control the spacing of the single-layer arrangement of droplets by controlling the pressure of the spacer oil at two sides. In the droplet detection conduit 25 after the "cross structure", after the droplets are spaced apart, they are lined up in a stream for optical detection.

The droplets passing through the above-mentioned cross structure are separated by the spacer oil, enter the droplet detection conduit 25, pass through the droplet detection conduit 25, and then enter the waste liquid tank 19 provided on the lower end of the lower surface of the first component 1 through the waste liquid port 26 of the droplets.

### Example 2 A droplet detection chip of the present invention

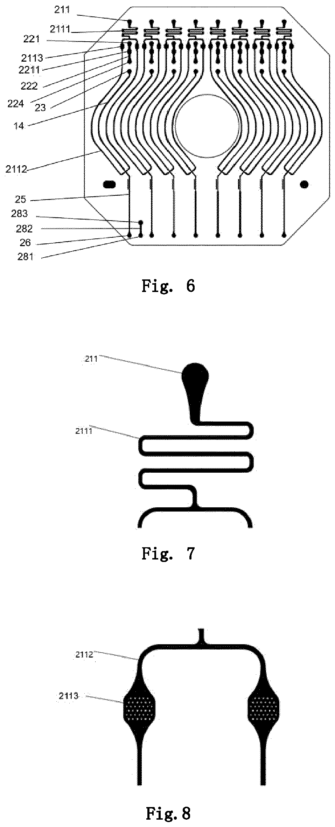

As shown in Figure 6, in a standard optical disc with an outer diameter of 118mm and an inner diameter of 22mm, eight identical droplet detection units are arranged at equal intervals in a standard disc from left to right for parallel fluorescence detection of droplets.

There is a central hole in the center of the chip. The central hole comes from the optical disc processing process, used for injection molding materials and substrate transfer during mass production, and for droplet detection system transfer during batch fluorescence detection. The traditional circular disc structure is difficult to locate. The chip is processed into an octagonal structure and two positioning holes are processed to facilitate the positioning and coordination of the droplet chip and related equipment. Four identical droplet detection units are arranged at equal intervals on both sides of the central hole, and are used to detect droplets in parallel.

As shown in Fig. 6, each droplet detection unit 1 includes, from top to bottom, a spacer oil inlet 211, a return flow resistance zone 2111, two spacer oil conduits 2112, two spacer oil filter zones 2113, floating oil inlet 221, floating oil filter zone 2211, a floating oil conduit 222 and a floating oil hole 224, a droplet floating hole 23, a droplet conduit 24, a droplet detection conduit 25 and a droplet waste liquid lid 26; floating oil hole 224 is a through hole connecting the floating oil conduit 222 and the third component 3 (not shown below). The droplet detection system shown in FIG. 1 revises the standard structure of a conventional optical disc, which can maximize the use of the disc space and arrange the droplet detection channels in parallel. At the same time, the chip processed by precision injection molding technology, combined with the design of the return flow resistance zone and filter zone, can quickly and reliably detect uniform micron-level "water-in-oil" droplets with fluorescence.

In one embodiment, in the eight droplet detection units in Fig. 6, the distance between each of the spacer oil inlets 211, the distance between the floating oil inlets 221, and the distance between the droplet floating holes 23, are equal, the distance is equal to the distance between standard eight-channel pipette tips.

As shown in Fig. 7, firstly, an external air pump or a peristaltic pump is used, and the spacer oil is injected into the spacer oil inlet 211. In order to precisely control the amount of loading spaced oil, in one embodiment, a return flow resistance zone 2111 is provided to precisely control the amount of loading spaced oil. The spacer oil will wet the surface of the polymer material. Under the condition of no pressure, the capillary oil will automatically flow into the micro-conduit. In extreme cases, the spacer oil flows continuously under the action of capillaries. The purpose of designing the return flow resistance zone 2111 is to accurately control the amount of loading spaced oil, and to minimize the continuous flow of the spaced oil in the micro conduit under the action of capillary, so that the amount of loading spaced oil is controlled only by an external air pump or peristaltic pump.

Then, the spacer oil passes through an oil phase split inlet and is divided into two into the same spacer oil conduit 2112. Its function is to make spacer oil droplet and floating oil droplet meet at the intersection and promote the movement of droplets. The flow effect squeezes the floating oil droplet to the center of the flow channel, which is convenient for detecting the fluorescent signal in the droplet. As shown in Fig. 8, each of the two spacer oils enters spacer oil filter zone 2113, respectively. The spacer oil filter zone 2113 is a group of columnar array structures. As shown in Fig. 8, the columnar array structure is composed of multiple rows of columnar arrays. Impurities (particles, flock fibers, etc.) present in the spacer oil are blocked at this group of columnar structures, thus, eliminating the effect of impurities on fluorescence detection of droplets.

Under the action of external air pressure, the floating oil flows from the floating oil inlet 221 into the floating oil conduit 222, flows down at the floating oil hole 224, and flows into the third component 3, while the droplets in the third component 3 are affected by the floating oil, floating from the droplet floating hole 23. The third component 3 is placed below the floating oil hole 224 and the droplet floating hole 23. The distance between the floating oil hole 224 and the droplet floating hole 23 is designed with reference to the size of the third component 3. If the third component 3 is an EP tube, the distance between the floating oil hole 224 and the droplet floating hole 23 is smaller than the width of the EP tube.

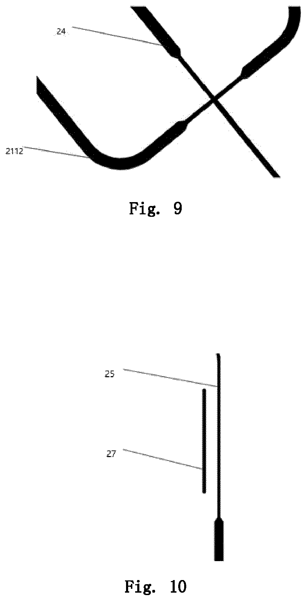

As shown in fig. 9, the spacer oil conduits 2112 and one droplet conduit 24 form a cross structure, and the purpose is to make the closely-spaced droplets floating up to be separated by the spacer oil on both sides, and to make signals interference between the droplets is reduced; at the same time, by the means of the "cross structure" design, the spacing of the single-layer arrangement of droplets can be controlled by controlling the pressure of the spacer oil at the two sides. In the droplet detection conduit behind the "cross structure", after the droplets are spaced apart, they flow in a line for optical detection.

As shown in fig. 10, after the "cross structure", the droplets have been arranged in a single row at a uniform interval in the droplet detection conduit, and a closed auxiliary channel 27 is set at a fixed distance beside the droplet detection conduit to be detected. There are no oil phase, water phase, or droplet samples flowing in the closed channel. The imaging, light transmission and astigmatism properties of this area are stable, which facilitates the positioning of the optical path detection system to the center of the droplet detection conduit 25.

With the detection of a large number of droplets, the droplets after the detecting signal flow out from the droplet waste liquid port 26 and flow into an external closed collection tank inlet. The purpose of the collection tank closure is to prevent cross-contamination caused by droplet waste. As shown in Fig. 5, in some embodiments, the droplet detection system is provided with an exhaust inlet 281, an exhaust line 282, and an exhaust outlet 283 of a collection tank, and the exhaust inlet 281 is connected to the closed collection tank outlet, and it is connected to the exhaust outlet 283 through the exhaust line 282, in order to release the pressure generated by the continuous inflow of liquid in the collection tank, while reducing cross-contamination.

It should be understood that the disclosed invention is not limited to the particular methods, technical solutions, and materials described, as these can vary. It should also be understood that the terminology used herein is for the purpose of describing particular embodiments only, and is not intended to limit the scope of the invention, which is limited only by the appended claims.

## Claims

1. A droplet detection apparatus, wherein said droplet detection apparatus comprises a first component, a second component, and a third component, said first component is the component used for loading spacer oil and floating oil when detecting droplets and collecting waste liquid after the detection of the droplets, said second component is a droplet detection chip, and said third component is a container holding droplets for droplet PCR amplification; and said second component is fixedly connected to the upper surface of said first component; and said third component is detachably fixedly connected to the lower surface of said first component.

2. The droplet detection apparatus according to claim 1, wherein said first component and said second component are respectively formed by integral injection molding.

3. The droplet detection apparatus according to claim 1, wherein said first component is provided with a locking slot for fixing said third component.

4. The droplet detection apparatus according to claim 3, wherein said third component comprises a droplet container and a cover of said droplet container, said droplet container is made of a rigid plastic material, and said cover is made of a soft plastic material; when fixing, said cover cooperates with said locking slot so that said first component and said third component are detachably fixedly connected .

5. The droplet detection apparatus according to claim 4, wherein said locking slot is provided with a puncture needle protruding from the lower surface of said first component, and said puncture needle is hollow; and when said first component and said third component are detachably fixedly connected, said puncture needle penetrates the cover of said droplet container.

6. The droplet detection apparatus according to claim 1, wherein said first component comprises at least one unit for loading spacer oil and floating oil when detecting droplets and collecting waste liquid after the detection of the droplets, preferably four, eight or twelve; said second component comprises at least one droplet detection unit, preferably four, eight or twelve; said third component comprises at least one said container, preferably four, eight, or twelve; one said unit for loading spacer oil and floating oil when detecting droplets and collecting waste liquid after the detection of the droplets, one said droplet detection unit and one said container holding droplets for droplet PCR amplification are matched to finish the detection of the droplets after PCR amplification.

7. The droplet detection apparatus according to claim 6, wherein said unit for loading spacer oil and floating oil when detecting droplets and collecting waste liquid after the detection of the droplets comprises a floating oil loading tank, a floating oil loading through hole, a spacer oil loading tank and a spacer oil loading through hole provided on the upper end of the upper surface of said first component; said floating oil loading through hole and said spacer oil loading through hole are respectively provided at the bottom of said floating oil loading tank and said spacer oil loading tank; and said floating oil loading through hole is connected to a floating oil injection conduit at the bottom of the lower surface of said first component, said spacer oil loading through hole is connected to a spacer oil injection conduit at the bottom of the lower surface of said first component.

8. The droplet detection apparatus according to claim 7, wherein a waste liquid tank is provided on the lower end of the lower surface of said first component and is used to collect all waste liquid after detection.

9. The droplet detection apparatus according to claim 8, wherein a configuration for discharging residual air in said waste liquid tank is provided on the lower end of said second component.

10. The droplet detection apparatus according to claim 1, wherein said droplet detection chip comprises one central hole, said central hole is used for injecting molding materials during the preparation of said droplet detection chip and used for transport of droplet detection chips during mass production of said droplet detection chips and/or mass fluorescence detection of said droplet detection chips; and one or more droplet detection units are provided on both sides of said central hole, each droplet detection unit independently detects droplets.

11. The droplet detection apparatus according to claim 10, wherein said droplet detection unit comprises a droplet detection conduit, a spacer oil inlet, a spacer oil conduit, a droplet container, a droplet floating hole and a droplet conduit; spacer oil enters said spacer oil conduit from said spacer oil inlet, and droplets enter said droplet conduit from said droplet container through said droplet floating hole; said spacer oil conduit and said droplet conduit form a cross structure before said droplet detection conduit, so that the droplets in said droplet conduit are separated by spacer oil.

12. The droplet detection apparatus according to claim 11, wherein a return flow resistance zone is provided in the conduit after said spacer oil inlet, and spacer oil is divided into two paths to enter two said spacer oil conduits after passing through said return flow resistance zone, respectively.

14. The droplet detection chip according to claim 11, wherein a spacer oil filter zone is provided in said spacer oil conduit and/or a floating oil filter zone is provided in said floating oil conduit, and said spacer oil filter zone and/or said floating oil filter zone are a set of columnar array structures, respectively.

15. The droplet detection chip according to claim 11, wherein said spacer oil conduit and / or said droplet conduit are arc-shaped conduit structures far from said central hole; an auxiliary channel is provided beside said droplet detection conduit, so that the optical path of detection system is positioned to the center of said droplet detection conduit.
